# EUROPEAN PATENT APPLICATION

(11) **EP 4 407 555 A1**
(43) Date of publication of application: **31.07.2024**
(21) Application number: 23153386.0
(22) Date of filing: 26.01.2023
(51) Int. Cl.: G06T 7/00, A61B 5/02

(54) **ESTIMATING DIFFUSE PLAQUE BURDEN OF A VESSEL**

(71) Applicant: Koninklijke Philips N.V., 5656 AG Eindhoven (NL)
(72) Inventor: WISSEL, Tobias, Eindhoven (NL); HAASE, Hans Christian, Eindhoven (NL); GRASS, Michael, 5656AG Eindhoven (NL)
(74) Representative: Philips Intellectual Property & Standards

(57) **Abstract**

There is provided a computer-implemented method of estimating a diffuse plaque burden of a vessel. The computer-implemented method comprises receiving S1 an angiogram 1000 including the vessel; identifying S2 a lumen of the vessel in the angiogram; approximating S3 a healthy lumen of the vessel based on determining a change in the lumen along a length of the vessel; and estimating S4 the diffuse plaque burden of the vessel based on subtracting the approximation of the healthy lumen from the lumen.

## Description

### FIELD OF THE INVENTION

The invention relates to a computer-implemented method of estimating a diffuse plaque burden of a vessel.

### BACKGROUND TO THE INVENTION

Vascular disease manifests itself in various forms. A particular risk of, for example, coronary artery disease (CAD) is that it may lead to myocardial infarction, ischemia and death.

A typical recommended diagnostic analysis in a stable patient is a pressure measurement over one or multiple localized lesions of a vessel to categorize the blood flow impairment caused by the one or multiple localized lesions. The result of this diagnostic analysis is used to decide whether a stent should be placed or not. However, such analyses do not fully represent the overall disease burden of the vessel.

Hence, there is a need for an improved method of analyzing the disease burden of a vessel. More specifically, there is a need for a method of analyzing the disease burden of a vessel that accounts for the diffuse disease burden. As alluded to, the diffuse disease burden is underrepresented in typical clinical scores. However, the diffuse disease burden an important marker of the systemic extent and future progression of vascular disease. It is an aim of the subject-matter of the present disclosure to improve on the prior art and address such problems.

### SUMMARY OF THE INVENTION

The subject-matter of the present disclosure provides a computer-implemented method which at least partially obviates or mitigates at least some of the disadvantages of the prior art, whether identified herein or elsewhere, or to provide an alternative approach. For instance, it is an aim of embodiments of the invention to provide a method of analyzing the disease burden of a vessel that accounts for the diffuse disease burden.

According to the present disclosure there is provided a computer-implemented method and a transitory or non-transitory computer-readable medium, as set forth in the appended claims. Other features of the invention will be apparent from the dependent claims and the description that follows.

According to a first aspect, there is provided a computer-implemented method of estimating a diffuse plaque burden of a vessel. The computer-implemented method comprises receiving an angiogram including the vessel; identifying a lumen of the vessel in the angiogram; approximating a healthy lumen of the vessel based on determining a change in the lumen along a length of the vessel; and estimating the diffuse plaque burden of the vessel based on subtracting the approximation of the healthy lumen from the lumen.

The angiogram may comprise a plurality of pixels. Identifying the lumen of the vessel in the angiogram may comprise identifying pixels of the plurality of pixels which define the lumen.

The method may further comprise generating a spatial domain signal of dimensional variations along the length of the vessel. Approximating the healthy lumen may comprise extracting a low frequency content based on the spatial domain signal using a low-pass filter.

The method may further comprise generating a spatial domain signal of dimensional variations along the length of the vessel. Approximating the healthy lumen may comprise applying the spatial domain signal to an isotonic regression model to fit a line of isotonic regression.

The method may further comprise generating a spatial domain signal of dimensional variations along the length of the vessel. Approximating the healthy lumen may comprise applying the spatial domain signal to a component analysis, CA, model trained to determine an eigenvector that relates healthy lumens to lumens in which diffuse plaque has been introduced.

The method may further comprise generating a spatial domain signal of dimensional variations along the length of the vessel. Approximating the healthy vessel may comprise applying the spatial domain signal to a neural network trained to determine a spatial domain signal of a healthy lumen of a vessel from a spatial domain signal of a lumen of a vessel.

The neural network may have been trained using spatial domain signals of paired images of healthy lumens and lumens in which diffuse plaque has been introduced.

Identifying the lumen of the vessel may comprise determining a center line of the lumen; mapping the center line onto a longitudinal axis; and correcting for any foreshortening of the lumen by resampling the lumen along the longitudinal axis.

The method may further comprise applying a local lesion identifying model configured to identify local lesions in the angiogram; and removing the local lesions from the angiogram.

The method may further comprise identifying a branching point in the lumen; and segmenting the lumen to remove the branching point.

The method may further comprise applying a signal windowing function to the lumen to remove artifacts caused by sudden changes to lumen geometry.

Identifying the lumen in the angiogram may comprise identifying the lumen based on at least one of: a computer tomography, CT, scan and an intravascular ultrasound scan, IVUS.

Estimating the diffuse plaque burden of the vessel may be further based on: calculating a metric representative of the diffuse plaque burden of the vessel, the metric being at least one of: a spectral power; a variance of the estimated plaque burden for intervals along the vessel; a standard deviation of the estimated plaque burden for intervals along the vessel; an absolute value of the estimated plaque burden; and a proportion of the vessel having an estimated plaque burden above a predetermined value.

The method may further comprise outputting the estimation of the diffuse plaque burden to a user interface.

The change in the lumen may be a change in one of: a radius, a diameter and a cross-sectional area.

Receiving an angiogram may comprise receiving a plurality of angiograms. Identifying a lumen of the vessel in the angiogram may comprise identifying the lumen of the vessel in the plurality of angiograms.

The vessel may be a coronary vessel.

According to a second aspect, there is provided a transitory, or non-transitory, computer-readable medium having instructions stored thereon which, when executed by a processor, cause the processor to carry out the method of the first aspect.

These and other aspects of the present invention will be apparent from and elucidated with reference to the embodiment(s) described hereinafter.

### BRIEF DESCRIPTION OF THE DRAWINGS

For a better understanding of the invention, and to show how embodiments of the same may be brought into effect, reference will be made, by way of example only, to the accompanying diagrammatic Figs, in which:
Figs 1A and 1B each show a flowchart of a computer-implemented method of estimating a diffuse plaque burden of a vessel, according to an embodiment;
Fig. 2 shows an angiogram of a vessel having a diffuse plaque burden (Dinakar Bootla, Pruthvi C Revaiah, Navjyot Kaur, Yash Paul Sharma, Himanshu Gupta, The Use of Complementary Technologies in Calcified Left Main Disease: A Case Series, Journal of Asian Pacific Society of Cardiology 2022;1:e13.);
Figs 3A and 3B respectively schematically depict a cross-section of a healthy lumen of a vessel and a cross-section of a lumen of the vessel in having a diffuse plaque burden, according to an embodiment;
Fig. 4 shows identification of contours on two angiograms and corresponding stretched lumen views;
Fig. 5 shows a graph depicting a change in a dimension of a lumen along a length of a vessel and an approximation of a change in the dimension of a healthy lumen of the vessel; and
Figs 6A and 6B respectively show perspective views of a vascular tree and the vascular tree in which a local lesion has been introduced.

### DETAILED DESCRIPTION

At least some of the example embodiments described herein may be constructed, partially or wholly, using dedicated special-purpose hardware. Terms such as 'component', 'module' or 'unit' used herein may include, but are not limited to, a hardware device, such as circuitry in the form of discrete or integrated components, a Field Programmable Gate Array (FPGA) or Application Specific Integrated Circuit (ASIC), which performs certain tasks or provides the associated functionality. In some embodiments, the described elements may be configured to reside on a tangible, persistent, addressable storage medium and may be configured to execute on one or more processors. These functional elements may in some embodiments include, by way of example, components, such as software components, object-oriented software components, class components and task components, processes, functions, attributes, procedures, subroutines, segments of program code, drivers, firmware, microcode, circuitry, data, databases, data structures, tables, arrays, and variables. Although the example embodiments have been described with reference to the components, modules and units discussed herein, such functional elements may be combined into fewer elements or separated into additional elements. Various combinations of optional features have been described herein, and it will be appreciated that described features may be combined in any suitable combination. In particular, the features of any one example embodiment may be combined with features of any other embodiment, as appropriate, except where such combinations are mutually exclusive. Throughout this specification, the term "comprising" or "comprises" means including the component(s) specified but not to the exclusion of the presence of others.

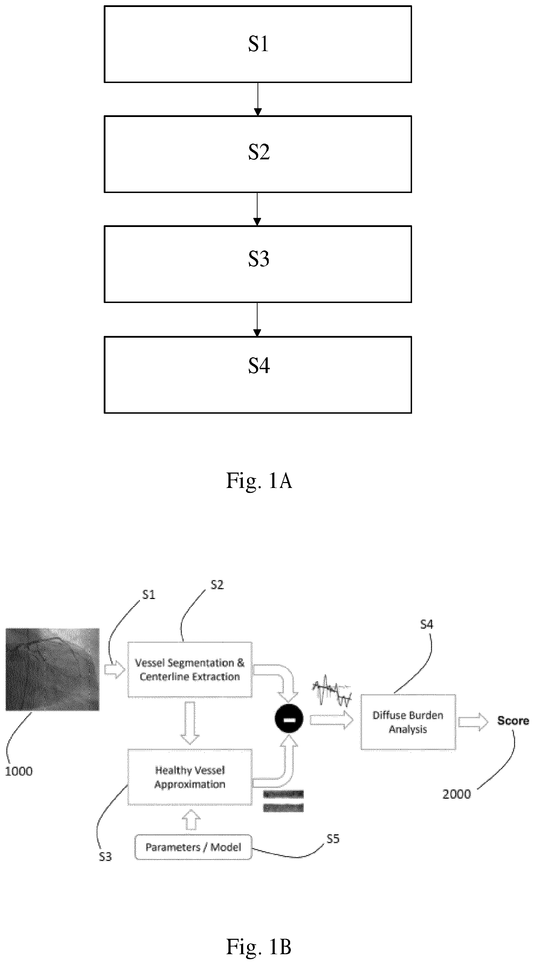

Fig. 1A shows a flowchart a computer-implemented method of estimating a diffuse plaque burden of a vessel (e.g., a coronary vessel), according to an embodiment. As shown in Fig. 1A, the method comprises receiving S1 an angiogram including the vessel; identifying S2 a lumen of the vessel in the angiogram; approximating S3 a healthy lumen of the vessel based on determining a change in the lumen along a length of the vessel (e.g., a change in a radius, a diameter, a cross-sectional area or X-ray intensity along the vessel, which can serve as a proxy for vessel thickness in the projection direction); and estimating S4 the diffuse plaque burden of the vessel based on subtracting the approximation of the healthy lumen from the lumen.

Fig. 1B shows a flowchart of the same computer-implemented method. As described above in relation to Fig. 1A, the method comprises receiving S1 at least one angiogram 1000 including the vessel. As shown in Fig. 1B and as discussed further below, identifying S2 a lumen of the vessel in the angiogram may comprise determining a center line. As described above in relation to Fig. 1A, the method comprises approximating S3 a healthy lumen of the vessel. As indicated in Fig. 1B, approximating S3 a healthy lumen of the vessel includes performing S5 various parameterization and modelling, which is described in detail below. As shown in Fig. 1B, estimating S4 the diffuse plaque burden of the vessel is based on subtracting the approximation of the healthy lumen from the lumen. As indicated in Fig. 1B and as described in more detail below, the estimated diffuse plaque burden may be represented as a score or metric 2000.

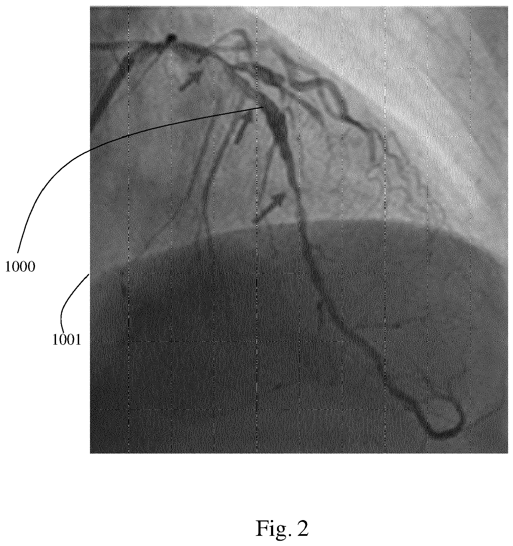

An angiogram is a two-dimensional image, obtained by taking an X-ray of a vessel filled with contrast medium. An example of an angiogram 1000 is shown in Fig. 2. The angiogram 100 shown in Fig. 2 shows a vessel 1001 having a diffuse plaque burden. Advantageously, angiograms provide a fine-scale resolution that is particularly suited to estimating the diffuse burden, because plaque variation on a higher spatial frequency level is not smoothened out by the imaging process.

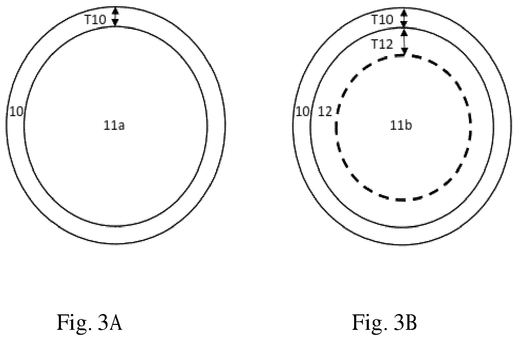

The term "lumen" is used in the conventional sense to refer to the interior space of a vessel through which blood typically flows. What is meant by the term "lumen," and how the diffuse plaque burden of the vessel of Fig. 1A is estimated can be further understood with the aid of Figs 3A and 3B.

Figs 3A and 3B respectively schematically depict a cross-section of a healthy lumen 11a of a vessel and a cross-section of a lumen 11b of the vessel having a diffuse plaque burden, according to an embodiment. In Fig. 3A, the healthy lumen 11a is defined by the vessel wall 10, which has a thickness T10. In Fig. 3B, the lumen 11b is defined by the vessel wall 10, having the thickness T10, and plaque 12, having a thickness T12. Consequently, the thickness T12 of the plaque is equivalent to the difference between the dimensions (e.g., radius, diameter, cross-sectional area) of the healthy lumen 11a and the dimensions (e.g., radius, diameter, cross-sectional area) of the lumen 11b with a diffuse plaque burden. In other words, as underlined by comparing Figs 3A and 3B, by subtracting the dimensions of the lumen 11b from the healthy lumen 11a, the diffuse plaque burden can be determined. Therefore, if the healthy lumen of a diseased vessel (i.e., a vessel having a diffuse plaque burden) is approximated, the diffuse plaque burden of the vessel in question can be estimated through this subtraction.

Receiving S1 an angiogram 1000, as shown in Fig. 1B, may comprise receiving a plurality of angiograms 1000. Thus, identifying S2 the lumen of the vessel in the angiogram 1000 may comprise identifying the lumen of the vessel in the plurality of angiograms 1000. Advantageously, using a plurality of angiograms 1000 may facilitate more accurate estimation of the diffuse plaque burden by eliminating, for example, artifacts resulting from different cardiac activity. The angiogram(s) 1000 may comprise a plurality of pixels such that identifying S2 the lumen of the vessel in the angiogram 1000 may comprise identifying pixels of the plurality of pixels which define the lumen.

Identifying S2 the lumen of the vessel may comprise determining a center line of the lumen. For example, in the case that the angiogram comprises a plurality of pixels, those pixels defining the interface between the vessel wall and the lumen may be identified. Subsequently, the center line of the lumen can be identified through the relative positions of the pixels corresponding to the interface between the vessel wall and the lumen. For instance, in the case of the lumen 11b shown in Fig. 3B, the center line of the lumen 11b is equidistant from each of the pixels identified as defining the interface between the plaque and the lumen 11b. In other words, the center line of the lumen 11b is the longitudinal axis of the cylinder having a cross-section corresponding to the dashed line defining the lumen 11b. Identifying the lumen of the vessel may be performed using standard image processing, a neural network or a combination thereof.

Subsequent to determining the center line of the lumen, identifying S2 the lumen may comprise mapping the center line onto a longitudinal axis. That is, the center line may be resampled/morphed into a stretched two-dimensional version in which the formerly sinuous centerline is mapped to a straight longitudinal axis (stretched lumen view). By stretching the lumen along a (straight) longitudinal axis, a better representation of the vessel is realized in the event of any foreshortening of the lumen that may be a result of an angle at which the X-ray was performed to generate the angiogram, for instance.

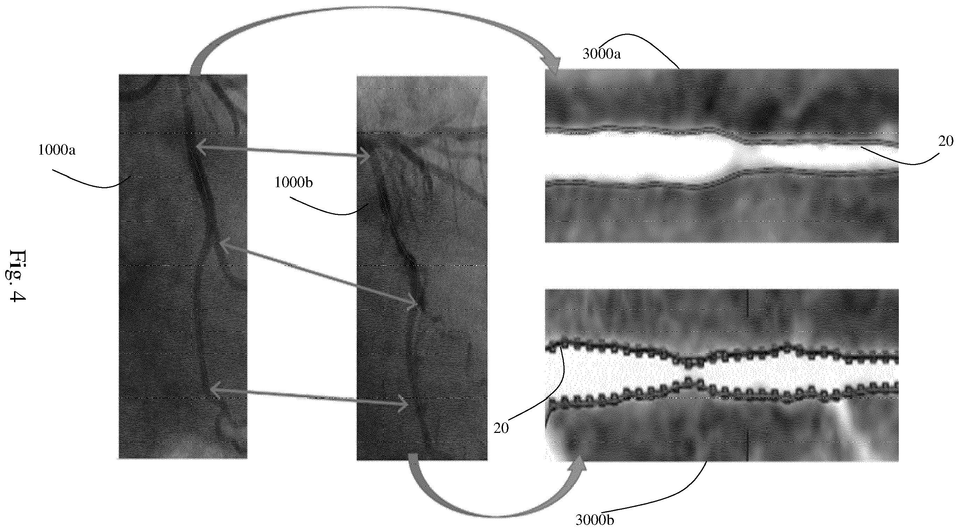

Fig. 4 shows center lines determined in two angiograms 1000a, 1000b. Adjacent to the angiograms 1000a, 1000b are shown corresponding stretched lumen views 3000a, 3000b of the vessels.. Lines 20 in Fig. 4 indicate contours of the interface between the vessel wall and any plaque with the lumens.

Identifying S2 the lumen in the angiogram may comprise identifying the lumen based on one or more of a computer tomography, CT, scan and an intravascular ultrasound scan, IVUS. By combining different inputs, advantageously, more accurate estimation of the diffuse plaque burden may be facilitated.

The method typically includes generating a spatial domain signal of dimensional variations along the length of the vessel. Advantageously, by generating a spatial domain signal of dimensional variations along the length of the vessel, the healthy lumen can be approximated, as detailed in the four examples below.

In a first example, approximating S3 the healthy lumen comprises extracting a low frequency content based on the spatial domain signal using a low-pass filter. Low frequency content is representative of the diffuse plaque burden because local lesions, for instance, are associated with high frequency content due to their relatively sparse distribution along the vessel. In this first example, extracting the low frequency content may comprise converting the spatial domain signal to a frequency domain signal. Typically, the spatial domain signal is converted to the frequency domain signal by applying a Fourier transformation to the spatial domain signal. However, the low frequency content may also be extracted by applying the low-pass filter in the spatial domain directly, for example, in the case of a finite impulse response (FIR) filter or an infinite impulse response (IIR) filter. Use of a low-pass filter is, advantageously, a computationally efficient way by which to approximate the healthy lumen. The output from the filter is a frequency domain or time domain signal corresponding to only low frequency components of the input signal. The low frequency components correspond to an approximation of the healthy lumen. If a frequency domain signal is output, an inverse Fourier transform may be performed if a spatial domain signal is desired.

In a second example, approximating S3 the healthy lumen comprises applying the spatial domain signal to an isotonic regression model to fit a line of isotonic regression. Isotonic regression is also known as monotonic regression. As with linear regression models, isotonic regression models seek to minimize the mean-squared error. However, unlike linear regression models, isotonic regression models are freeform. In other words, isotonic regression models fit a piecewise-constant, monotonic function to data. In this second example an isotonic regression model is applied to the spatial domain signal, thereby fitting a line of isotonic regression to the spatial domain signal such that the change in the lumen along the vessel is determined, the change in the lumen along the vessel serving as an approximation of the healthy vessel. The line of isotonic regression is the output from the isotonic regression model. The line of isotonic regression is an approximation of a healthy lumen. Advantageously, as alluded to, isotonic regression is not constrained by a particular functional form.

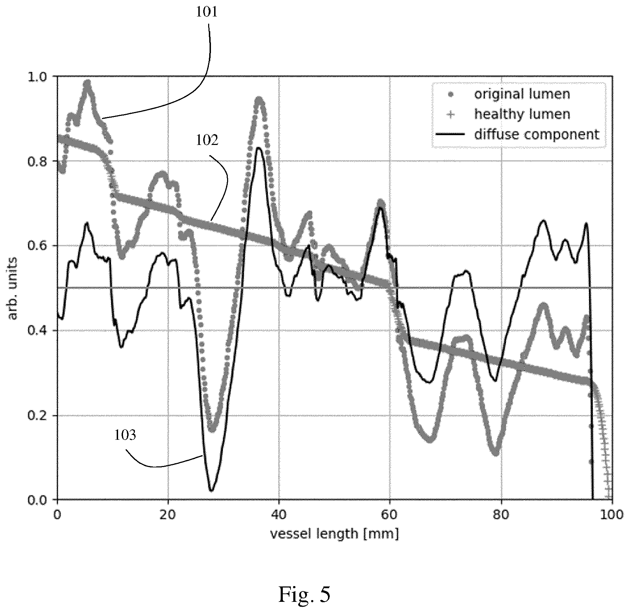

Fig. 5 shows a graph depicting a change in a dimension of a lumen of along a length of a vessel (line 101) and an approximation of a change in the dimension cross-sectional area of a healthy lumen of the vessel (line 102). The x-axis shows the length of the vessel in millimeters, and the y-axis shows signal intensity for the aforementioned dimensions (e.g., radius, diameter, cross-sectional area) in arbitrary units. In Fig. 5, the approximation of the cross-sectional area of the healthy lumen of the vessel has been determined by fitting an isotonic line of regression. The thin line 103 shows the difference between the observed lumen signal 101 and the approximated healthy lumen signal 102 by means of isotonic regression. The thin line 103, therefore, corresponds to the residual signal which can be considered the (raw - i.e., effects from local lesions, branching points, etc, may present) diffuse plaque component.

In a third example, approximating S3 the healthy lumen comprises applying the spatial domain signal to a component analysis (e.g., PCA or ICA), model trained to determine at least one component that relates healthy lumens to lumens in which diffuse plaque has been introduced. PCA, for instance, is a method that reduces the dimensionality of large data sets. PCA reduces the dimensionality of large data sets by transforming a large set of variables into a smaller one that still contains most of the information in the large data set. PCA advantageously reduces the number of variables, hence the simplicity and computational expense of any associated analysis, while minimizing any loss of accuracy.

Performing PCA involves determining the covariance matrix for a set of variables, in this case, variables describing the change in the lumen along the length of the vessel. If the eigenvectors and eigenvalues of the covariance matrix are computed, the percentage contribution to the variance of the data by each of the variables can be determined, meaning that the variables containing the least information can be discarded to leave the principal component(s). The variables that account for the greatest percentage of variation of the data are typically those which provide the most accurate approximation of a healthy lumen (e.g., change in lumen diameter). Therefore, by training the PCA model using information (e.g., angiograms) pertaining to unhealthy lumens, at least one eigenvector can be obtained that corresponds to the variable that accounts for the greatest contribution to the variance of the data (principal component). In this way, one or more eigenvectors are determined that can be used to approximate a healthy lumen given a lumen comprising diffuse plaque. Referring to Fig. 5, the input to the PCA model may be considered information derived from angiograms that corresponds to the line 101 (i.e., a change in a dimension of a lumen of along a length of a vessel), while determination of the eigenvector enables output of information corresponding to line 102 (i.e., approximation of a change in the dimension of a lumen along the length of a healthy vessel). In other words, the signal corresponding to the lumen of an unhealthy vessel in input to the PCA model and the output form the model is a signal corresponding to an approximation of a healthy lumen.

In a fourth example, approximating S3 the healthy vessel comprises applying the spatial domain signal of dimensional variations along the length of the vessel to a neural network trained to determine a spatial domain signal of dimensional variations along the length of a given vessel having a healthy lumen from a spatial domain signal of dimensional variations along the length of the given vessel having diffuse plaque. The neural network may have been trained using spatial domain signals derived from images of healthy lumens and spatial domain signals of lumens in which diffuse plaque has been introduced. In the latter case, the diffuse plaque, and other lesions, may be introduced synthetically using an adversarial agent, for example. In other words, the neural network is trained to generate a spatial domain signal approximating contours of a healthy vessel burden (e.g., information corresponding to line 102 in Fig. 5) from a spatial domain signal corresponding to contours of a vessel of a patient's lumen that has lesions including a diffuse plaque burden (e.g., information corresponding to line 101 in Fig. 5). Consequently, for a given vessel, the neural network is able to approximate a healthy lumen of the vessel.

Estimating S4 the diffuse plaque burden of the vessel includes subtracting the signal corresponding to the approximation of the healthy lumen from the signal corresponding to the vessel of the lumen (see line 103 in Fig. 5, which shows an estimated diffuse plaque burden). Further, the estimating S4 the diffuse plaque burden of the vessel may include calculating one or more metrics representative of the diffuse plaque burden of the vessel using the signal resulting from the subtraction. In other words, estimating the diffuse plaque burden S4 may include computing a quantity that describes the estimated diffuse plaque burden using the signal resulting from the subtraction. Advantageously, calculating a metric representative of the estimated diffuse plaque burden may engender ease of comparison between the condition of vessels (e.g., between patients) or facilitate tracking of patient history.

In one example, following subtracting the approximation of the healthy lumen from the lumen to obtain an estimated absolute diffuse plaque burden, the estimated absolute diffuse plaque burden may be considered in intervals along the vessel (e.g., intervals of a predetermined width, such as a particular percentage of the total length of the vessel). Thus, by summing the estimated diffuse plaque burden of each interval and by dividing this summation by the number intervals, an estimated mean diffuse plaque burden can be calculated. The number and width of the intervals may be adjustable. From the estimated mean diffuse plaque burden, an estimated variance of the diffuse plaque burden and an estimated standard deviation of the diffuse plaque burden may be straightforwardly calculated.

In another example, a proportion of the vessel having an estimated plaque burden above a predetermined value may be calculated. The predetermined may be a value that has been established as medically significant (e.g., an estimated diffuse plaque burden that is considered to have a deleterious effect on health). For instance, if the estimated absolute diffuse plaque burden is considered in intervals along the vessel, a number of intervals having an estimated diffuse plaque burden above the predetermined value can be calculated. For example, if the estimated absolute diffuse plaque burden is considered for the vessel divided into 100 intervals, it may be that 70 of these 100 intervals have an estimated diffuse plaque burden below the predetermined value and 30 of these intervals have an estimated diffuse plaque burden equal to or above the predetermined value. Therefore, the proportion of the vessel having an estimated diffuse plaque burden above the predetermined value is 30, which may be indicative of the vessel being in poor health.

In another example, the spectral power of the estimated diffuse plaque burden may be calculated by applying a Fourier transformation to the signal corresponding to the estimated diffuse plaque burden and by integrating the signal along the length of the vessel.

The method may comprise outputting the estimation of the diffuse plaque burden to a user interface of, for example, a tablet, smart phone, laptop or alike, meaning that the metric is conveniently viewable by a medical practitioner. The user interface may be configured to display the estimated diffuse plaque burden on top of the vessel in the angiogram received. Similarly, the user interface may be configured to enable highlighting of particular vascular segments (e.g., those with an estimated diffuse plaque burden above a predetermined threshold).

The method may further comprise applying a signal windowing function to the lumen to remove artifacts caused by sudden changes to lumen geometry. A signal windowing function is a function that is zero-valued outside a particular range, typically symmetric about the middle of the range. Sudden changes to lumen geometry (e.g., sudden changes in lumen diameter) may be caused by, for example, localized lesions (as opposed to diffuse lesions/plaque),which generally cause sudden narrowing of the lumen. Sudden changes to lumen geometry may mean that the lumen is not well behaved, which may be associated with branching points. Therefore, advantageously, by applying the signal windowing function, artifacts that may worsen the accuracy of the estimation of the diffuse plaque burden are discarded.

In the same way that the signal windowing function may be applied to the lumen to remove artifacts caused by sudden changes to lumen geometry, the method may further comprise applying a local lesion identifying model configured to identify local lesions (i.e., localized or focal lesions) in the angiogram and removing the local lesions from the angiogram. The local lesion identifying model may be, for example, a neural network trained using paired images of healthy lumens and lumens in which local lesions have been introduced. Advantageously, compared with applying the signal windowing function, applying the local lesion identifying model may more accurately eliminate localized lesions from the estimation of the diffuse plaque burden, resulting in more accurate estimation of the diffuse plaque burden.

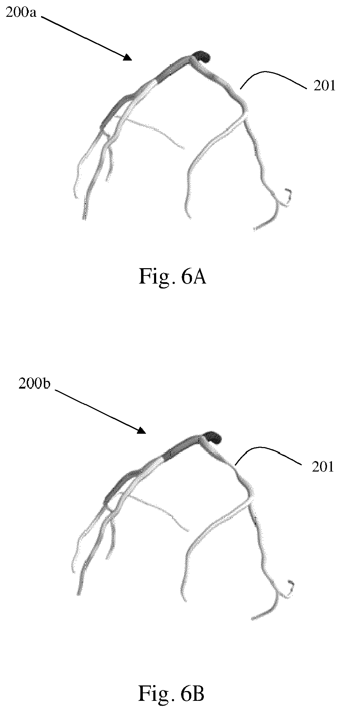

Figs 6A and 6B respectively show a vascular tree 200a (without a local lesion) and the same vascular tree 200b in which a local lesion has been introduced. These vascular trees 200a, 200b have been synthetically generated. Figs 6A and 6B indicate a position 201 where the local lesion has been synthetically introduced in Fig. 6B.

Similarly, the method may further comprise identifying a branching point in the lumen and segmenting the lumen to remove the branching point. A branching point is a point at which a vessel diverges into at least one other vessel. For example, the coronary blood vessels branch into smaller vessels that penetrate the muscle mass to supply blood to the entire heart. Branching points may be identified based on sudden changes in lumen geometry (e.g., diameter) and/or direction, for example. Advantageously, compared with applying the signal windowing function, specifically identifying and removing branching points may more accurately eliminate these parts of the vessel from the estimation of the diffuse plaque burden, resulting in more accurate estimation of the diffuse plaque burden.

In summary, the invention provides a computer-implemented method that enables improved analysis the disease burden of a vessel, in particular the diffuse plaque burden. Consequently, systemic extent and future progression of vascular disease can be better understood.

The optional features set out herein may be used either individually or in combination with each other where appropriate and particularly in the combinations as set out in the accompanying claims. The optional features for each aspect or exemplary embodiment of the invention, as set out herein, are also applicable to all other aspects or exemplary embodiments of the invention, where appropriate. In other words, the skilled person reading this specification should consider the optional features for each aspect or exemplary embodiment of the invention as interchangeable and combinable between different aspects and exemplary embodiments.

All of the features disclosed in this specification (including any accompanying claims and drawings), and/or all of the steps of any method or process so disclosed, may be combined in any combination, except combinations where at most some of such features and/or steps are mutually exclusive.

Although a preferred embodiment has been shown and described, it will be appreciated by those skilled in the art that various changes and modifications might be made without departing from the scope of the invention, as defined in the appended claims and as described above.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measured cannot be used to advantage. Any reference signs in the claims should not be construed as limiting the scope.

## Claims

1. A computer-implemented method of estimating a diffuse plaque burden of a vessel, the computer-implemented method comprising:
receiving (S1) an angiogram (1000) including the vessel;
identifying (S2) a lumen of the vessel in the angiogram;
approximating (S3) a healthy lumen of the vessel based on determining a change in the lumen along a length of the vessel; and
estimating (S4) the diffuse plaque burden of the vessel based on subtracting the approximation of the healthy lumen from the lumen.

2. The method of claim 1, further comprising:
generating a spatial domain signal of dimensional variations along the length of the vessel,
wherein approximating the healthy lumen comprises extracting a low frequency content based on the spatial domain signal using a low-pass filter.

3. The method of claim 1, further comprising:
generating a spatial domain signal of dimensional variations along the length of the vessel,
wherein the approximating the healthy lumen comprises applying the spatial domain signal to an isotonic regression model to fit a line of isotonic regression.

4. The method of claim 1, further comprising:
generating a spatial domain signal of dimensional variations along the length of the vessel,
wherein approximating the healthy lumen comprises applying the spatial domain signal to a component analysis, CA, model trained to determine an eigenvector that relates healthy lumens to lumens in which diffuse plaque has been introduced.

5. The method of claim 1, further comprising:
generating a spatial domain signal of dimensional variations along the length of the vessel,
wherein approximating the healthy vessel comprises applying the spatial domain signal to a neural network trained to determine a spatial domain signal of a healthy lumen of a vessel from a spatial domain signal of a lumen of the vessel.

6. The method of claim 5, wherein the neural network has been trained using spatial domain signals derived from paired images of healthy lumens and lumens in which diffuse plaque has been introduced.

7. The method of any preceding claim, wherein identifying the lumen of the vessel comprises:
determining a center line of the lumen;
mapping the center line onto a longitudinal axis; and
correcting for any foreshortening of the lumen by resampling the lumen along the longitudinal axis.

8. The method of any preceding claim, further comprising:
applying a local lesion identifying model configured to identify local lesions in the angiogram; and
removing the local lesions from the angiogram.

9. The method of any preceding claim, further comprising:
identifying a branching point in the lumen; and
segmenting the lumen to remove the branching point.

10. The method of any preceding claim, further comprising:
applying a signal windowing function to the lumen to remove artifacts caused by sudden changes to lumen geometry.

11. The method of any preceding claim, wherein the estimating the diffuse plaque burden of the vessel is further based on:
calculating a metric representative of the diffuse plaque burden of the vessel, the metric being at least one of: a spectral power; a variance of the estimated plaque burden for intervals along the vessel; a standard deviation of the estimated plaque burden for intervals along the vessel; an absolute value of the estimated plaque burden; and a proportion of the vessel having an estimated plaque burden above a predetermined value.

12. The method of any preceding claim, further comprising:
outputting the estimation of the diffuse plaque burden to a user interface.

13. The method of any preceding claim, wherein the change in the lumen is a change in one of: a radius, a diameter and a cross-sectional area.

14. The method of any preceding claim, wherein the vessel is a coronary vessel.

15. A transitory, or non-transitory, computer-readable medium having instructions stored thereon which, when executed by a processor, cause the processor to carry out the method of any preceding claim.
